(19) Europäisches Patentamt
European Patent Office
Office européen des brevets

(11) Veröffentlichungsnummer: **0 205 400**
**A2**

(12) # EUROPÄISCHE PATENTANMELDUNG

(21) Anmeldenummer: 86810229.4

(22) Anmeldetag: 28.05.86

(51) Int. Cl.[4]: C 07 D 301/02
C 07 D 303/04

(30) Priorität: 03.06.85 US 740445
27.01.86 US 822994

(43) Veröffentlichungstag der Anmeldung:
17.12.86 Patentblatt 86/51

(84) Benannte Vertragsstaaten:
AT BE CH DE FR GB IT LI NL

(71) Anmelder: CIBA-GEIGY AG
Klybeckstrasse 141
CH-4002 Basel(CH)

(72) Erfinder: Coers, Klaus Jürgen
Lochackerstrasse 10
CH-4153 Reinach(CH)

(72) Erfinder: Radimerski, Paul, Dr.
Brüelrainweg 6
CH-4147 Aesch(CH)

(54) **Verfahren zur Herstellung von Alkyloxiranen.**

(57) Ein neues Verfahren zur Herstellung von Alkyloxiranen der Formel

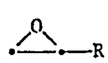

worin R für $C_1$-$C_{12}$-Alkyl steht, durch Umsetzung eines aliphatischen Aldehyds der Formel

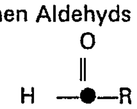

worin R für $C_1$-$C_{12}$-Alkyl steht, mit einem Sulfonium-Salz der Formel

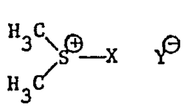

worin X für $C_1$-$C_{18}$-Alkyl und $Y^{\ominus}$ für ein Gegenanion stehen, in Gegenwart einer wässrigen Alkalihydroxidlösung ist dadurch gekennzeichnet, dass man die Reaktion in der Weise durchführt, dass man die Alkalihydroxidlösung vorlegt, den Aldehyd und das Sulfonium-Salz gleichzeitig in äquimolaren Mengen zufügt und als Gegenanion das Methosulfatanion $H_3CO$-$SO_2$-$O^{(-)}$ wählt.

EP 0 205 400 A2

CIBA-GEIGY AG
Basel (Schweiz)

5-15359/CIP/=

## Verfahren zur Herstellung von Alkyloxiranen

Die vorliegende Erfindung betrifft ein neues Verfahren zur Herstellung von Alkyloxiranen aus aliphatischen Aldehyden durch Behandlung mit Schwefelyliden.

Die nach dem neuen Verfahren herstellbaren Alkyloxirane sind wertvolle Zwischenprodukte für die Synthese von 1,2-Dihydroxyalkanen, welche ihrerseits Zwischenprodukte für hochwirksame Pflanzenmikrobizide und Pflanzenwuchsregulatoren aus der Klasse der 2-Phenyl-2-triazolyl-methyl-dioxolane darstellen. 2-Phenyl-2-triazolylmethyldioxolane, ihre Herstellung und Verwendung sind beispielsweise im US-Patent 4.079.062 beschrieben. Als wichtiger Vertreter dieser Stoffklasse ist das 2-(2,4-Dichlorphenyl)-2-(1H-1,2,4-triazol-1-ylmethyl)-4-n-propyl-dioxolan zu nennen, das unter der Bezeichnung Propiconazol bekannt ist. Es kann durch Umsetzung von ω-Brom-2,4-dichloracetophenon mit 1,2-Pentandiol zum 2-Brommethyl-2-(2,4-dichlorphenyl)-4-n-propyl-dioxolan und dessen weitere Umsetzung mit 1H-1,2,4-Triazol hergestellt werden. Die Hydrolyse der erfindungsgemäss herstellbaren Alkyloxirane der Formel I zu den entsprechenden 1,2-Dihydroxyalkanen erfolgt nach üblichen Methoden in Gegenwart von katalytischen Mengen einer anorganischen Säure oder Base.

Aus der Literatur sind Synthesemethoden zur Herstellung von Oxiranen durch Methylid-Addition an eine Carbonylgruppe seit langem bekannt. Als geeignete Reagenzien für die Oxiran-Synthese werden Dimethyloxosulfonium-methylid und Dimethylsulfoniummethylid von Corey und Chaykovsky (J. Amer. Chem. Soc. 87/6, 1353-1364, 1965) beschrieben. Die bisher beschriebenen Synthesemethoden eignen sich nur ungenügend für grosstechnische Umsetzungen, da einerseits ungenügende Ausbeuten erzielt werden oder andererseits die Verfahren nur für aromatische

Aldehyde oder Ketone anwendbar sind (Deutsche Offenlegunsschriften 3315524 und 3315619). Andere Verfahren sind unwirtschaftlich oder ökologisch bedenklich, weil teure und/oder umweltbelastende Reagenzien eingesetzt werden müssen (Vgl. Helv. Chim. Acta 63 (6), 1980, 1665-1674, US-PS 3,442,912 und EP-A-94 726).

Es besteht somit ein Bedürfnis nach einem kostengünstigen, in grosstechnischem Massstab durchführbaren Verfahren zur Herstellung von Alkyloxiranen aus aliphatischen Aldehyden.

Ueberraschenderweise befriedigt das erfindungsgemässe neue Verfahren dieses Bedürfnis weitgehend.

Das erfindungsgemässe Verfahren zur Herstelllung von Alkyloxiranen der Formel I

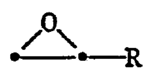 (I)

worin R für $C_1$-$C_{12}$-Alkyl steht, durch Umsetzung eines aliphatischen Aldehyds der Formel II

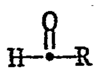 (II)

worin R für $C_1$-$C_{12}$-Alkyl steht, mit einem Sulfonium-Salz der Formel III

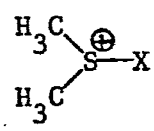 $Y^{\ominus}$ (III)

worin X für $C_1$-$C_{18}$-Alkyl und $Y^{\ominus}$ für ein Gegenanion stehen, in Gegenwart einer wässrigen Alkalihydroxidlösung, ist dadurch gekennzeichnet, dass man die Reaktion in der Weise durchführt, dass man die Alkalihydroxidlösung vorlegt, den Aldehyd und das Sulfonium-Salz gleichzeitig in äquimolaren Mengen zufügt und als Gegenanion das Methosulfatanion $H_3CO$-$SO_2$-$O^{\ominus}$ wählt.

Der gleichzeitige Zusatz von Aldehyd und Sulfonium-Salz kann entweder vorzugsweise aus zwei getrennten synchron arbeitenden Dosiervorrichtungen erfolgen oder in Form einer einzigen Lösung, die sowohl

den Aldehyd als auch das Sulfonium-Salz enthält.

Ueblicherweise findet die Reaktion in einer zweiphasigen Reaktionsmischung statt, die aus einer wässrigen und einer mit Wasser nicht mischbaren organischen Phase besteht. Die organische Phase besteht hauptsächlich aus den beiden Reaktanden und dem resultierenden Endprodukt. Vorteilhafterweise wird die Reaktion in einem inerten Lösungsmittel durchgeführt. In bestimmten Fällen, besonders bei kontinuierlicher Verfahrensführung, kann das Verfahren auch ohne Lösungsmittel durchgeführt werden. Die im erfindungsgemässen Verfahren verwendeten inerten Lösungsmittel sind nicht mit Wasser mischbar. Geeignete Lösungsmittel sind für diesen Zweck: aromatische Lösungsmittel wie Benzol, Toluol, Xylol, Nitrobenzol, Nitrotoluol oder vorzugsweise die verschiedenen Halogenbenzole. Als Lösungsmittel geeignete Halogenbenzol-Derivate sind 2-Chlortoluol, 3-Chlortoluol, 4-Chlortoluol, Chlorbenzol, ortho-Dichlorbenzol, meta-Dichlorbenzol und para-Dichlorbenzol, vorzugsweise aber Chlorbenzol. Aus arbeitstechnischen Gründen wird zweckmässigerweise das Alkalihydroxid in Form einer konzentrierten wässrigen Lösung verwendet, während die Reaktionspartner der Formeln II und III als Lösungen im organischen Lösungsmittel eingesetzt werden. Zweckmässig ist auch die Verwendung von Lösungen der Formeln II und III, in denen der Lösungsmittelanteil über 50 % liegt.

In den literaturbekannten Oxiransynthesen unter Verwendung eines Sulfoniumsalzes, bzw. des in situ gebildeten Schwefelylids, werden verschiedene Gegenanionen verwendet, wie Halogenanionen, vorzugsweise $Br^{\ominus}$ oder $J^{\ominus}$, Carbonatanionen, Nitratanionen, Sulfatanionen, Acetatanionen, Perchloratanionen oder Tosylatanionen. Im erfindungsgemässen Verfahren hat sich überraschenderweise das Methosulfatanion $H_3CO-SO_2-O^{\ominus}$ als hervorragend geeignet erwiesen.

Durch den Einsatz einer wässrigen Alkalihydroxid-Lösung und der reinen Reaktanden II und III oder von Lösungen derselben in organischen, mit Wasser nicht mischbaren Lösungsmitteln ergeben sich die erwähnten zweiphasigen Reaktionsmischungen.

Wahlweise können der zweiphasigen Reaktionsmischung auch übliche Phasen-Transfer-Katalysatoren zugesetzt werden. Diese Massnahme zeigt nur geringe Auswirkungen auf die erzielbare Ausbeute an gewünschtem Alkyloxiran. Uebliche Phasen-Transfer-Katalysatoren sind zum Beispiel quaternäre Ammoniumsalze wie Trimethylbenzylammoniumbromid, Trimethylbenzylammoniumchlorid, Triäthylbenzylammoniumchlorid, Triäthylbenzylammoniumbromid, Tetrabutylammoniumchlorid oder Tributylmethylammoniumbromid; oder Kronenäther wie 18-Crown-6, 15-Crown-5 oder 12-Crown-4.

Die Alkylgruppen, die unter die Definitionen von R und X fallen, umfassen alle möglichen isomeren $C_1$-$C_{18}$-Formen. Bevorzugt werden im erfinderischen Verfahren jedoch die geradkettigen Kohlenwasserstoffreste eingesetzt. R steht vorzugsweise für $C_2$-$C_6$-Alkyl, insbesondere aber für n-Propyl. X steht vorzugsweise für $C_5$-$C_{12}$-Alkyl, insbesondere aber für n-Hexyl. Insbesondere ist das erfindungsgemässe Verfahren mit Vorteil anzuwenden, wenn gleichzeitig R für $C_2$-$C_6$-Alkyl und X für $C_5$-$C_{12}$-Alkyl stehen.

Die Reaktionspartner der Formeln II und III werden in dem erfindungsgemässen Verfahren in ungefähr gleichen molaren Mengen eingesetzt, wobei geringe Ueberschüsse der Komponente II bis zu 20 Mol % keinen nennenswerten Einfluss auf die erzielbare Ausbeute am Produkt der Formel I hat. Die wässrige Alkalihydroxidlösung wird in mindestens gleichen molaren Mengen eingesetzt, wie das Sulfonium-Salz der Formel III. Bevorzugt ist ein Ueberschuss an Alkalihydroxid bis zu 20 Mol an Alkalihydroxid pro Mol Sulfonium-Salz. Ganz besonders bevorzugt ist der Einsatz von 1 bis 10 Mol Alkalihydroxid pro Mol Sulfonium-Salz. Uebliche Alkalihydroxide sind Natriumhydroxid und Kaliumhydroxid. Für den erfindungsgemässen Prozess wird mit Vorteil Kaliumhydroxid eingesetzt. Unreagiertes Kaliumhydroxid kann rezykliert werden.

Die Reaktionstemperaturen liegen im allgemeinen zwischen 10°C und 140°C. Um eine möglichst hohe Produktausbeute und Reinheit zu erhalten, hat sich eine Verfahrensführung bei Reaktionstemperaturen zwischen 60°C und 100°C als besonders zweckmässig erwiesen. Wird die Reaktion in dem angegebenen Temperaturbereich durchgeführt, können die Reaktionszeiten zwischen 10 Minuten und 2 Stunden gehalten werden. Vorzugsweise beträgt die Reaktionsdauer zwischen 20 und 40 Minuten. Die genannten Reaktionsbedingungen beziehen sich auf eine Arbeitsweise unter Normaldruck. Wegen der einfachen Verfahrenshandhabung wird die Reaktionsführung unter Normaldruck bevorzugt, jedoch ist bisher kein Nachteil für die Durchführung bei niedrigeren oder höheren Drücken zwischen 10 mbar und 20 bar festgestellt worden.

Das erfindungsgemässe Verfahren kann sowohl bei diskontinuierlicher als auch kontinuierlicher Reaktionsführung eingesetzt werden. In beiden Fällen ist jedoch stets auf die gleichzeitige Zugabe der Reaktionspartner II und III zur vorgelegten Alkalihydroxidlösung zu achten.

In einer bevorzugten Ausführungsform des erfindungsgemässen Verfahrens führt man die Reaktion in einem Gemisch von Chlorbenzol und Wasser bei einer Temperatur zwischen 60°C und 100°C aus.

In ganz besonderer Weise werden die Vorteile des neuen Verfahrens offenbar, wenn man in einem Gemisch von Chlorbenzol und Wasser bei einer Temperatur zwischen 60°C und 100°C Butyraldehyd mit Dimethylhexylsulfonium-methosulfat in Gegenwart von Kalumhydroxid umsetzt.

Die nachfolgenden Beispiele illustrieren das neue erfindungsgemässe Verfahren zur Herstellung von Alkyloxiranen.

Beispiel 1: 2-Propyloxiran

In einem Glaskolben mit Rührwerk legt man 800 g 50 %ige (10 Mol) wässrige Natriumhydroxidlösung bei einer Temperatur von 80°C vor. Dazu lässt man unter kräftigem Rühren innerhalb von 30 Minuten eine Lösung von 516 g (2 Mol) Dimethylhexylsulfonium-methosulfat und 158,4 g (2,2 Mol) Butyraldehyd in 516 g Chlorbenzol zutropfen. Während dieser Zeit wird die Temperatur bei 80°C gehalten. Nach weiteren 15 Minuten wird die Reaktionsmischung abgekühlt und mit 2 l Wasser verdünnt. Die organische Phase wird abgetrennt, über Magnesiumsulfat getrocknet und an einer Füllkörperkolonne fraktioniert. Man erhält so 136 g 2-Propyloxiran von 95 %iger Reinheit (Ausbeute: 75 % d.Th.), Sdp. 88 - 92°C.

Beispiel 2: 2-Propyloxiran

Eine Rührreaktorkaskade von 0,5 l Gesamtvolumen wird mit gemäss Beispiel 1 erhaltenen Reaktionsmischung gefüllt und auf 80°C temperiert. Innerhalb von 2 Stunden werden gleichmässig und in gleichen Molverhältnissen 800 g (10 Mol) 50 %ige Natriumhydroxidlösung sowie eine Mischung aus 516 g (2 Mol) Dimethylhexylsulfoniummethosulfat, 151,2 g (2,1 Mol) Butyraldehyd und 516 g Chlorbenzol zudosiert. Die mittlere Verweilzeit in der Rührreaktorkaskade beträgt 30 Minuten. Die überlaufende Reaktionslösung wird mit Wasser verdünnt. Die organische Phase wird abgetrennt, mit Magnesiumsulfat getrocknet und an einer Füllkörperkolonne fraktioniert. Man erhält so 145 g 2-Propyloxiran von 95 %iger Reinheit (Ausbeute: 80 % d.Th.), Sdp. 88 - 92°C.

Beispiel 3: 2-Propyloxiran

Bei einer Reaktionsführung in einer Rührreaktorkaskade von 0.5 l Gesamtvolumen werden bei einer Temperatur von 80°C 280 g 50 %ige (2.5 Mol) Kaliumhydroxidlösung vorgelegt und innerhalb von 30 Minuten unter kräftigem Rühren tropfenweise mit einer Mischung aus 129 g (0,5 Mol) Dimethylhexylsulfonium-methosulfat, 37,8 (0,525 Mol) Butyraldehyd und

129 g Chlorbenzol auf das Gesamtvolumen von 0,5 l aufgefüllt. Anschliessend werden in 2 Stunden in konstantem Molverhältnis weitere 1120 g 50 %ige (10 Mol) Kaliumhydroxidlösung und eine Mischung aus 516 g (2 Mol) Dimethylhexylsulfonium-methosulfat, 151,2 g (2,1 Mol) Butyraldehyd und 516 g Chlorbenzol zudosiert. Die überlaufende Reaktionslösung wird wie in den Beispielen 1 und 2 aufgearbeitet. Ausbeute: 145 g 95 %iges (80 % d.Th.) 2-Propyloxiran, Sdp. 88 - 92 °C.

Beispiel 4: 2-Propyloxiran

In einem Glaskolben mit Rührwerk legt man 800 g 50 %ige (10 Mol) wässrige Natriumhydroxidlösung bei einer Temperatur von 80°C vor. Dazu lässt man unter kräftigem Rühren innerhalb von 30 Minuten eine gerührte Mischung von 516 g (2 Mol) Dimethylhexylsulfonium-methosulfat und 158,4 g (2,2 Mol) Butyraldehyd in 516 g Xylol zutropfen. Während dieser Zeit wird die Temperatur bei 80°C gehalten. Nach weiteren 15 Minuten wird die Reaktionsmischung abgekühlt und mit 2 l Wasser verdünnt. Die organische Phase wird abgetrennt, über Magnesiumsulfat getrocknet und an einer Füllkörperkolonne fraktioniert. Man erhält so 127 g 2-Propyloxiran von 95 %iger Reinheit (Ausbeute: 70 % d.Th.), Sdp. 88 - 92°C.

Beispiel 5: 2-Propyloxiran

Wie im Beispiel 3 wird eine Rührreaktorkaskade von 0,5 l Gesamtvolumen mit einer 50 %igen wässrigen Kaliumhydroxidlösung, Dimethylhexylsulfonium-methosulfat, Butyraldehyd und Chlorbenzol gefüllt. Anschliessend werden die Komponenten Kaliumhydroxid, Dimethylhexylsulfonium-methosulfat und Butyraldehyd gleichzeitig in einem molaren Verhältnis von 5 : 1 : 1,05 solange zugesetzt, bis in dem überlaufenden Reaktionsgemisch kein Chlorbenzol mehr nachweisbar ist. Anschliessend werden weitere 1120 g (10 Mol) 50 %iger wässriger Kaliumhydroxidlösung, 516 g (2 Mol) Dimethylhexylsulfonium-methosulfat und 151,2 g (2,1 Mol) Butyraldehyd gleichzeitig über einen Zeitraum von 2 Stunden zudosiert.

Die überfliessende Reaktionmischung wird analog zum Beispiel 2 aufgearbeitet. Man erhält 127 g (70 % d. Th.) 2-Propyloxiran von 95 % Reinheit.

Patentansprüche:

1. Verfahren zur Herstellung von Alkyloxiranen der Formel I

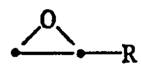

(I)

worin R für $C_1$-$C_{12}$-Alkyl steht, durch Umsetzung eines aliphatischen Aldehyds der Formel II

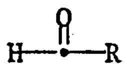

(II)

worin R für $C_1$-$C_{12}$-Alkyl steht, mit einem Sulfonium-Salz der Formel III

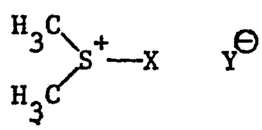

(III)

worin X für $C_1$-$C_{18}$-Alkyl und $Y^{\ominus}$ für ein Gegenanion stehen,in Gegenwart einer wässrigen Alkalihydroxidlösung, dadurch gekennzeichnet, dass man die Reaktion in einem Gemisch eines Halogenbenzols mit Wasser in der Weise durchführt, dass man die Alkalihydroxidlösung vorlegt, den Aldehyd und das Sulfonium-Salz gleichzeitig in äquimolaren Mengen zufügt und als Gegenanion das Methosulfatanion $H_3CO$-$SO_2$-$O^{\ominus}$ wählt.

2. Verfahren gemäss Anspruch 1, dadurch gekennzeichnet, dass man die Reaktion in einem inerten organischen aromatischen Lösungsmittel ausführt.

3. Verfahren gemäss Anspruch 2, dadurch gekennzeichnet, dass man die Reaktion in einem Gemisch eines Halogenbenzols mit Wasser ausführt.

//

4. Verfahren gemäss Anspruch 3, dadurch gekennzeichnet, dass man die Reaktion in einem Gemisch von Chlorbenzol und Wasser durchführt.

5. Verfahren gemäss Anspruch 1, dadurch gekennzeichnet, dass man eine Reaktionstemperatur zwischen 60°C und 100°C einhält.

6. Verfahren gemäss Anspruch 1, dadurch gekennzeichnet, dass R für $C_2$-$C_6$-Alkyl steht.

7. Verfahren gemäss Anspruch 6, dadurch gekennzeichnet, dass R für Propyl steht.

8. Verfahren gemäss Anspruch 1, dadurch gekennzeichnet, dass X für $C_5$-$C_{12}$-Alkyl steht.

9. Verfahren gemäss Anspruch 8, dadurch gekennzeichnet, dass X für Hexyl steht.

10. Verfahren gemäss Anspruch 1, dadurch gekennzeichnet, dass R für $C_2$-$C_6$ Alkyl und X für $C_5$-$C_{12}$-Alkyl stehen.

11. Verfahren gemäss Anspruch 1, dadurch gekennzeichnet, dass man die Reaktion in einem Gemisch von Chlorbenzol und Wasser bei einer Temperatur zwischen 60°C und 100°C ausführt.

12. Verfahren gemäss Anspruch 1, dadurch gekennzeichnet, dass man in einem Gemisch von Chlorbenzol und Wasser bei einer Temperatur zwischen 60°C und 100°C Butyraldehyd mit Dimethylhexylsulfoniummethosulfat in Gegenwart von Kaliumhydroxid umsetzt.